(19) Europäisches Patentamt / European Patent Office / Office européen des brevets

(11) **EP 4 667 977 A1**

(12) **DEMANDE DE BREVET EUROPEEN**

(43) Date de publication:
**24.12.2025 Bulletin 2025/52**

(21) Numéro de dépôt: **25180791.3**

(22) Date de dépôt: **04.06.2025**

(51) Classification Internationale des Brevets (IPC):
**G01T 1/167** (2006.01)    **G21C 17/06** (2006.01)

(52) Classification Coopérative des Brevets (CPC):
**G01T 1/167**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **18.06.2024 FR 2406476**

(71) Demandeur: **Commissariat à l'Energie Atomique et aux Energies Alternatives
75015 Paris (FR)**

(72) Inventeurs:
• **BOTTAU, Vincent
13115 SAINT-PAUL LEZ DURANCE (FR)**
• **ELEON, Cyrille
13115 SAINT-PAUL LEZ DURANCE (FR)**
• **PEROT, Bertrand
13115 SAINT-PAUL LEZ DURANCE (FR)**

(74) Mandataire: **Atout PI Laplace
Immeuble Up On
25 Boulevard Romain Rolland
CS 40072
75685 Paris Cedex 14 (FR)**

(54) **METHODE DE DETERMINATION D'UNE QUANTITE DE PLUTONIUM EN PRESENCE DE CURIUM PAR MESURE NEUTRONIQUE PASSIVE**

(57) Procédé de détermination d'une quantité de plutonium dans un échantillon radioactif en présence de curium, comprenant les étapes de :

- Mesurer (301) un train d'impulsions électriques au moyen d'un système de détection de radiations,

- Déterminer (302), respectivement dans une première et dans une seconde fenêtres temporelles, un premier et un second nombre de coïncidences d'impulsions d'ordre 2 entre les impulsions électriques fournies par les détecteurs,

- La seconde fenêtre temporelle étant constituée de l'union de la première fenêtre temporelle et d'une troisième fenêtre temporelle,

- La première fenêtre temporelle et la troisième fenêtre temporelle étant choisies de manière à ce que le nombre de coïncidences d'impulsions d'ordre 2 liées au plutonium soit plus important que celui lié au curium dans l'une des deux fenêtres parmi la première fenêtre temporelle et la troisième fenêtre temporelle et moins important que celui lié au curium dans l'autre fenêtre,

- Déterminer (304) la quantité de plutonium en appliquant un modèle de régression non linéaire prenant comme paramètres : le nombre total d'impulsions mesurées, le premier nombre de coïncidences d'impulsions d'ordre 2 et le second nombre de coïncidences d'impulsions d'ordre 2, le modèle étant au préalable entrainé sur un ensemble de valeurs simulées à partir d'un modèle numérique.

[Fig. 3]

FIG.3

**Description**

L'invention concerne le domaine de la mesure neutronique passive pour la caractérisation de déchets radioactifs ou de matières nucléaires (solides ou en solution). Plus précisément, elle concerne la mesure des coïncidences des neutrons et des rayonnements gamma issus de fissions spontanées pour la détermination de la masse de plutonium dans les colis de déchets radioactifs, les échantillons de matières nucléaires (lingots, pots de poudre, pastilles ou crayons de combustible, rebuts d'usinage, ou tout autre forme), ou encore les solutions de matières nucléaires (procédés de retraitement des combustibles usés).

La présence éventuelle, dans les colis de déchets radioactifs et matières nucléaires, de $^{242}$Cm et $^{244}$Cm peut entrainer une forte surestimation de la quantité de plutonium. En effet, pour prendre l'exemple du $^{244}$Cm qui est plus souvent présent (période radioactive de 18,1 ans) que le $^{242}$Cm qui disparait rapidement (période de 162 jours), l'émission neutronique spécifique (n.s$^{-1}$.g$^{-1}$) par fission spontanée du $^{244}$Cm est environ 10 000 fois plus grande que celle du $^{240}$Pu, principal émetteur neutronique du plutonium par fission spontanée. Par conséquent, la présence de curium dans un colis de déchets peut potentiellement masquer le signal neutronique issu de la fission spontanée des isotopes pairs du plutonium ($^{238,240,242}$Pu mais on ne parle que du principal émetteur $^{240}$Pu pour simplifier). Dans ce cas, l'évaluation de la masse totale de plutonium à partir de la masse équivalente de $^{240}$Pu (celle qui émettrait autant de neutrons que l'ensemble des isotopes $^{238,240,242}$Pu) devient très incertaine, avec un risque de surestimation très important, ce qui pose des problèmes à différents niveaux, par exemple pour vérifier un critère de sûreté-criticité ou pour la comptabilité des matières quand on s'intéresse à la masse fissile (isotopes $^{239\,et\,241}$Pu), ou pour la gestion des sites de stockages des déchets radioactifs (critères d'acceptation initiaux et activité alpha à long terme, notamment pour les isotopes $^{239\,et\,240}$Pu). Dans ce type de cas, il n'est pas envisageable d'utiliser des informations a priori (i.e. non mesurées, fournies par le détenteur des matières nucléaires ou le producteur des déchets) sur les proportions relatives de plutonium et de curium, car même une faible incertitude sur cette proportion massique induirait une erreur d'estimation trop importante de la quantité de plutonium.

Afin d'estimer précisément la masse de plutonium malgré la présence de curium dans les déchets radioactifs ou matières nucléaires, il existe deux types de méthodes de mesure : soit une mesure neutronique active, soit une mesure neutronique passive.

La mesure neutronique active est basée sur l'utilisation d'un générateur de neutrons pulsé. Cette méthode, décrite dans la référence [10] est actuellement la solution préconisée afin d'obtenir un rapport suffisamment élevé entre le signal (neutrons prompts de fissions induites sur le plutonium, mais aussi l'uranium) et le bruit de fond (passif principalement, dû aux fissions spontanées du curium). Cependant, la mise en œuvre d'une mesure active entraine des contraintes techniques et des coûts beaucoup plus importants que la mesure passive. En effet, le prix d'un générateur de neutrons est élevé et dépend du niveau d'émission nécessaire, de plus sa détention et son utilisation sont soumises à des autorisations réglementaires nécessitant notamment des formations spécifiques des opérateurs, des moyens de radioprotection (casemate d'irradiation) et de protection physique de l'appareil (bien à double usage), ainsi qu'une comptabilité pour la source de tritium qu'il contient.

Pour ces raisons, il est moins couteux de rechercher une méthode basée sur une mesure neutronique passive.

La très grande majorité des postes de mesure neutronique passive destinés à la caractérisation du plutonium (matières nucléaires, colis de déchets radioactifs, solutions de procédé, etc.), par la détection des neutrons de fission spontanée émis en coïncidence, est équipée de compteurs proportionnels gazeux à 3He car ils sont très sensibles aux neutrons thermiques et peu sensibles au rayonnement gamma.

Dans certains cas, la présence parasite de curium, émetteur très intense de neutrons par fission spontanée, conduit à surestimer de façon très significative la quantité de plutonium.

Actuellement, il existe deux principales méthodes de mesure neutronique passive mises en œuvre avec des détecteurs à 3He pour l'évaluation de la masse de plutonium en présence de curium, toutes deux basées sur le fait que les isotopes du curium émettent plus de neutrons par fission spontanée que ceux du plutonium (2,72 en moyenne contre 2,16, avec des distributions de multiplicités différentes voir référence [4]).

Ces méthodes sont basées sur l'exploitation de mesures de coïncidences d'impulsions à partir d'un train d'impulsions mesuré par les détecteurs. Une coïncidence d'impulsions d'ordre N ou supérieur, avec N un entier strictement positif, est détecté pour chaque impulsion lorsque, dans une fenêtre temporelle de taille donnée démarrant sur l'instant d'arrivée de l'impulsion, N-1 impulsions supplémentaires sont détectées dans la fenêtre temporelle.

Une première méthode d'évaluation par mesure neutronique passive consiste en l'évaluation du rapport entre le comptage des coïncidences d'impulsions réelles d'ordre 2 correspondant à des paires de neutrons et le comptage total des impulsions. Cette méthode, décrite dans la référence [5], est généralement utilisée pour les postes de mesure qui n'ont pas une efficacité de détection très élevée ou de système de dénombrement des multiplicités neutroniques.

Dans cette première méthode, une augmentation significative du ratio entre coïncidences d'impulsions réelles et comptage total peut être observée en présence de $^{244}$Cm. Or il se trouve que ce ratio évolue comme l'inverse de l'efficacité de détection apparente des neutrons. Par conséquent, toute diminution non maitrisée de cette efficacité apparente (comme par exemple dans le cas de colis de déchets contenant des éléments qui ralentissent puis absorbent les neutrons, ou qui contiennent uniquement des éléments modérateurs rendant les neutrons plus difficiles à détecter quand le poste de mesure contient un absorbant comme du cadmium devant les détecteurs), pourrait être assimilée à tort à une présence de curium. Ainsi, cette méthode ne permet généralement pas d'estimer précisément la quantité de plutonium en présence de curium et n'apporte qu'une information qualitative sur une possible présence de curium et le risque associé de majorer la masse de plutonium.

Une seconde méthode d'évaluation par mesure neutronique passive consiste en une mesure des multiplicités neutroniques et notamment du rapport entre le comptage des coïncidences d'impulsions réelles d'ordre 2 (appelées doublets) et celui des coïncidences d'impulsions réelles d'ordre 3 (appelées triplets). Cette seconde méthode est par exemple décrite dans les références [1] et [8].

Cette seconde méthode exploite également l'écart entre la distribution de probabilité du nombre de neutrons émis par fission spontanée du $^{244}$Cm et celle associée au $^{240}$Pu (les deux principaux émetteurs du Cm et du Pu). Le ratio entre les comptages des triplets et des doublets est plus élevé pour le $^{244}$Cm que pour le $^{240}$Pu, ce qui permet a priori de les discriminer. La référence [1] indique qu'il est envisageable d'évaluer la quantité de plutonium jusqu'à une fraction maximale en atomes de $^{244}$Cm de 1000 ppm, soit un rapport maximum entre les masses de $^{244}$Cm et de $^{240}$Pu de l'ordre de 0,1%. De plus, la mise en œuvre de cette méthode nécessite d'utiliser des postes de mesure neutronique équipés d'un très grand nombre de compteurs 3He (jusqu'à 5 couronnes de 40 compteurs 3He entourant un fût de déchets de 100 litres) afin d'obtenir des efficacités de détection (nombre de coups par neutron émis) très élevées, de 30% à 40%, et par conséquent des taux de comptage des triplets statistiquement exploitables. Mais dans la majorité des cas, notamment dans les colis de déchets radioactifs où les effets de matrice réduisent cette efficacité de détection et où le rapport entre les masses de Cm et de Pu peut atteindre plusieurs pourcents, cette méthode ne fournit comme précédemment qu'une information qualitative sur un risque de présence de $^{244}$Cm, comme indiqué également dans la référence [8].

La demande de brevet du Demandeur publiée sous le numéro EP 3 835 831 A1 concerne un procédé de mesure du plutonium par la détection passive des coïncidences neutroniques avec des scintillateurs plastiques PVT (polyvinyle toluène, détecteurs sensibles aux neutrons rapides) en remplacement des détecteurs à 3He, en raison principalement de leur coût trop élevé et de leur sensibilité aux coïncidences accidentelles (fenêtres de coïncidences beaucoup plus longues). La méthode proposée est basée sur une sélection temporelle de fenêtres de coïncidences en tenant compte, pour le comptage des triplets, des temps d'arrivée entre les trois particules détectées (neutrons et rayonnements gamma prompts) pour mieux discriminer les triplets dus aux fissions spontanées de ceux dus à diverses réactions parasites telles que les réactions ($\alpha$,n), mais aussi les diffusions élastiques et inélastiques créant de la diaphonie et donc des coïncidences indésirables. Cependant, cette méthode ne prend pas en compte les erreurs d'estimation liées à la présence du curium $^{244}$Cm.

Il existe donc un besoin pour une méthode permettant d'estimer avec précision la quantité de $^{240}$Pu en présence du $^{244}$Cm dans des matières nucléaires ou dans des fûts de déchets radioactifs, par mesure passive des coïncidences neutroniques, dans les cas où l'art antérieur ne le permettait pas, à savoir avec un rapport entre les masses de $^{244}$Cm et de $^{240}$Pu supérieur à 0,1 %. En outre il est nécessaire de s'affranchir des contributions gamma parasites dues aux produits de fission ou d'activation (comme le $^{137}$Cs, $^{60}$Co) potentiellement présents dans les objets à mesurer car les scintillateurs plastiques utilisés dans les détecteurs sont sensibles à ce type de rayonnements.

L'invention propose une nouvelle méthode de détermination de la masse de plutonium par mesure neutronique passive qui exploite la technique décrite dans la référence EP 3 835 831 A1 mais l'améliore en présence de curium.

Pour cela, l'invention est basée sur un modèle de régression non linéaire à plusieurs variables explicatives qui sont des nombres de coïncidences réelles de différents ordres comptabilisés dans deux intervalles temporels différents, dimensionnés de sorte que, dans l'un des deux intervalles, la contribution du plutonium est différente (plus élevée ou plus faible) que celle du curium, et dans l'autre intervalle, c'est l'inverse.

Dans la suite de la description, les nombres de coïncidences d'impulsions réelles d'ordre 2 sont aussi appelés doublets. Les nombres de coïncidences d'impulsions réelles d'ordre 3 sont aussi appelés triplets.

La prise en compte de deux intervalles temporels pour calculer les doublets et les triplets associés à l'entrainement d'un modèle de régression non linéaire paramétré par des variables explicatives calculées sur ces deux intervalles distincts, permet de mieux caractériser la masse de plutonium en présence de curium contrairement aux méthodes de l'art antérieur.

L'invention a pour objet un procédé de détermination d'une quantité de plutonium dans un échantillon radioactif en présence de curium, le procédé comprenant les étapes de :

- Mesurer un train d'impulsions électriques au moyen d'un système de détection de radiations comprenant plusieurs détecteurs disposés autour de l'échantillon et aptes à générer une impulsion électrique en réponse à la détection d'un rayonnement,

- Déterminer, respectivement dans une première et dans une seconde fenêtres temporelles, un premier et un second nombre de coïncidences d'impulsions d'ordre 2 entre les impulsions électriques fournies par les détecteurs,

- La seconde fenêtre temporelle étant constituée de l'union de la première fenêtre temporelle et d'une troisième fenêtre temporelle,

- La première fenêtre temporelle et la troisième fenêtre temporelle étant choisies de manière à ce que le nombre de coïncidences d'impulsions d'ordre 2 liées au plutonium soit plus important que celui lié au curium dans l'une des deux fenêtres parmi la première fenêtre temporelle et la troisième fenêtre temporelle et moins important que celui lié au curium dans l'autre fenêtre,

- Déterminer la quantité de plutonium en appliquant un modèle de régression non linéaire prenant comme paramètres : le nombre total d'impulsions mesurées, le premier nombre de coïncidences d'impulsions d'ordre 2 et le second nombre de coïncidences d'impulsions d'ordre 2, le modèle étant au préalable entrainé sur un ensemble de valeurs simulées à partir d'un modèle numérique.

Selon un aspect particulier de l'invention, la seconde fenêtre temporelle est choisie de manière à inclure des paires d'impulsions correspondant à des rayonnements de type ($\gamma$,n) et (n,n) et d'exclure des paires d'impulsions correspondant à des rayonnements de type ($\gamma$,$\gamma$).

Dans une variante de réalisation, le procédé selon l'invention comprend en outre les étapes de :

- Déterminer, respectivement dans la première et dans la seconde fenêtre temporelle, un premier et un second nombre de coïncidences d'impulsions d'ordre 3 entre les impulsions électriques fournies par les détecteurs,

- Le modèle de régression non linéaire prenant en outre comme paramètres lesdits premier et second nombres de coïncidences d'impulsions d'ordre 3.

Dans une variante de réalisation le procédé selon l'invention comprend en outre les étapes de :

- Déterminer un premier nombre de coïncidences d'impulsions d'ordre 3 entre les impulsions électriques fournies par les détecteurs qui est tel que l'écart temporel entre la première et la deuxième impulsions est compris dans la première fenêtre temporelle, et l'écart temporel entre la deuxième et la troisième impulsion est compris dans la première fenêtre temporelle,

- Déterminer un second nombre de coïncidences d'impulsions d'ordre 3 entre les impulsions électriques fournies par les détecteurs qui est tel que l'écart temporel entre la première et la deuxième impulsions est compris dans la deuxième fenêtre temporelle et l'écart temporel entre la deuxième et la troisième impulsion est compris dans la deuxième fenêtre temporelle,

- Le modèle de régression non linéaire prenant en outre comme paramètres lesdits premier et second nombres de coïncidences d'impulsions d'ordre 3.

Selon un aspect particulier de l'invention, la seconde fenêtre temporelle est choisie de manière à inclure des triplets d'impulsions correspondant à des rayonnements de type (y,n,n) et (n,n,n) et à exclure des triplets d'impulsions correspondant à des rayonnements de type (y,y,y).

Selon un aspect particulier de l'invention, la première fenêtre temporelle est égale à [10 ns ; 20 ns], la deuxième fenêtre temporelle est égale à [10 ns ; 60 ns] et la troisième fenêtre temporelle est égale à [20 ns ; 60 ns].

Selon un aspect particulier de l'invention, la première fenêtre temporelle est égale à [20 ns ; 60 ns], la deuxième fenêtre temporelle est égale à [10 ns ; 60 ns] et la troisième fenêtre temporelle est égale à [10ns ; 20 ns].

L'invention a aussi pour objet un système de détermination d'une quantité de plutonium dans un échantillon radioactif en présence de curium, le système comprenant plusieurs détecteurs disposés autour de l'échantillon et aptes à générer une impulsion électrique en réponse à la détection d'un rayonnement et un calculateur configuré pour exécuter les étapes du procédé selon l'invention.

Selon un aspect particulier de l'invention, chaque détecteur comprend un scintillateur organique plastique en polyvinyle-toluène (PVT).

D'autres caractéristiques et avantages de la présente invention apparaîtront mieux à la lecture de la description qui suit en relation aux dessins annexés suivants.

[Fig. 1] représente un premier schéma d'un système de mesure neutronique passive selon un mode de réalisation de l'invention,

[Fig. 2] représente un second schéma d'un système de mesure neutronique passive selon un mode de réalisation de l'invention,

[Fig. 3] représente un organigramme détaillant les étapes de mise en œuvre d'un procédé de détermination d'une quantité de plutonium selon un mode de réalisation de l'invention,

[Fig. 4a] représente un exemple de détermination de coïncidences pour un train d'impulsions,

[Fig. 4b] représente un tableau des distributions d'ordres de multiplicité pour l'exemple de la figure 4a,

[Fig. 5] représente un exemple de courbe de Rossi-Alpha,

[Fig. 6] représente un exemple de diagramme de spectre en énergie des neutrons de fission spontanée du $^{244}$Cm et du $^{240}$Pu,

[Fig. 7] représente les courbes de Rossi-Alpha respectives pour $^{244}$Cm et $^{240}$Pu,

[Fig. 8] illustre un premier exemple d'estimation d'une masse de $^{240}$Pu à l'aide d'un premier type de modèle de régression non linéaire,

[Fig. 9] illustre un premier exemple d'estimation d'une masse de $^{240}$Pu à l'aide d'un deuxième type de modèle de régression non linéaire,

[Fig. 10] illustre un premier exemple d'estimation d'une masse de $^{240}$Pu à l'aide d'un troisième type de modèle de régression non linéaire,

[Fig. 11] illustre un second exemple d'estimation d'une masse de $^{240}$Pu à l'aide du deuxième type de modèle de régression non linéaire,

[Fig. 12] illustre un autre exemple d'estimation d'une masse de $^{240}$Pu à l'aide d'un quatrième type de modèle de régression non linéaire,

[Fig. 13] illustre le calcul d'un nombre de coïncidences d'impulsion d'ordre 3 avec sélection temporelle à partir d'un histogramme en 2 dimensions selon un principe de l'art antérieur,

[Fig. 14] illustre un autre exemple d'estimation d'une masse de $^{240}$Pu à l'aide d'un cinquième type de modèle de régression non linéaire,

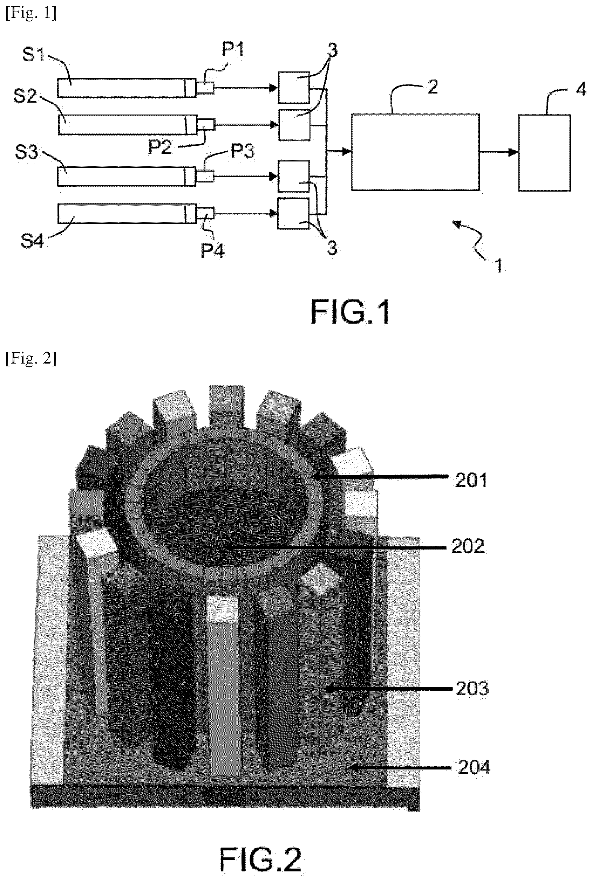

La figure 1 représente un schéma simplifié d'un système de mesure neutronique passive selon un mode de réalisation de l'invention.

Sur cet exemple non limitatif, le système de mesure comprend 4 détecteurs, chaque détecteur comprenant un scintillateur S1-S4 plastique PVT et un photomultiplicateur P1-P4. Le nombre de détecteurs peut être plus élevé, par exemple égal à 10 ou à 16.

Le système de mesure comprend également un calculateur 2 relié aux photomultiplicateurs P1-P4 par l'intermédiaire de convertisseurs analogique-numérique 3. Le système 1 peut également comprendre un module d'interface homme machine 4, par exemple un écran sur lequel sont restitués les résultats de mesure.

La figure 2 représente un autre exemple de schéma d'un système de mesure neutronique passive comprenant cette fois 16 détecteurs 203 basés sur des scintillateurs plastiques PVT. Le système comporte par ailleurs un écran de plomb 201 disposé sous forme d'un tube à l'intérieur des détecteurs et un fût 202 dans lequel est disposé un échantillon. Le système repose sur une palette support 204. Dans une variante de réalisation, l'écran de plomb 201 peut être supprimé ou son épaisseur peut être adaptée selon l'intensité des rayonnements d'émission gamma de l'échantillon à analyser.

Le système de mesure selon l'invention est équipé de scintillateurs plastiques PVT (sans discrimination neutron-gamma) tel que décrit dans la demande de brevet référencée [2].

Le système de mesure utilise préférentiellement des scintillateurs plastiques en polyvinyle-toluène PVT et non des détecteurs 3He pour les raisons suivantes.

L'utilisation de détecteurs 3He impose des constantes de temps de l'ordre de plusieurs dizaines de microsecondes qui correspondent à la durée nécessaire à la thermalisation des neutrons, contrairement aux scintillateurs plastiques qui détectent directement les neutrons rapides et pour lesquels les constantes de temps sont de l'ordre de la dizaine de nanosecondes. Cette propriété utilisée dans les références [2] et [3] pour limiter le nombre de coïncidences accidentelles et différencier le signal du plutonium de celui de sources parasites gamma (présence de produits de fission ou d'activation dans l'objet mesuré) et neutroniques (réactions ($\alpha$,n) sur des noyaux légers comme l'oxygène dans les oxydes de plutonium, en présence d'émetteurs alpha intenses comme l' $^{241}$Am ou le $^{238}$Pu).

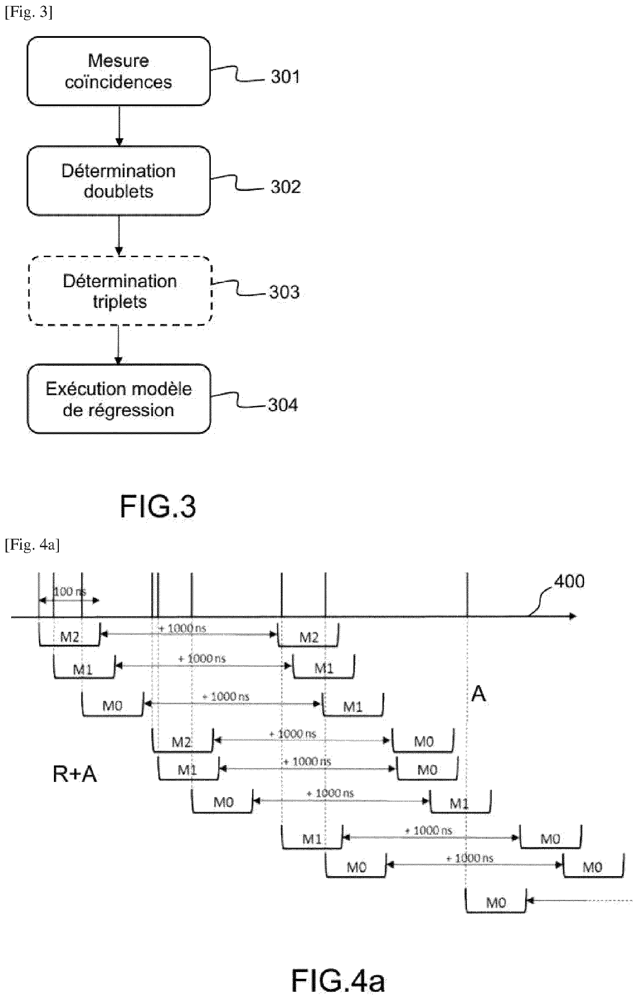

La figure 3 représente un organigramme détaillant les étapes de mise en œuvre d'un procédé de détermination d'une quantité de plutonium en présence de curium dans un échantillon au moyen du système décrit aux figures 1 et 2.

Le procédé débute à l'étape 301 par une étape de mesures de coïncidences réalisées au moyen du système décrit ci-dessus pour un échantillon donné. Chaque détecteur comprend un photomultiplicateur qui est apte à fournir un signal électrique représentatif du signal lumineux généré dans le scintillateur plastique. Ce signal électrique est enregistré pour chaque détecteur. A l'issue de cette étape de mesure, on obtient un train d'impulsions, chaque impulsion étant caractérisée au moins par son instant d'arrivée et son énergie de rayonnement.

A l'étape 302, on détermine, à partir du train d'impulsions mesuré à l'étape 301, un nombre de coïncidences d'ordre 2 pour deux fenêtres temporelles particulières dont le choix sera explicité plus loin.

A l'étape 303, on détermine optionnellement, également un nombre de coïncidences d'ordre 3 pour les mêmes fenêtres temporelles qu'à l'étape 302.

Enfin, à l'étape 304, on exécute un modèle de régression non linéaire qui est entrainé pour déterminer une quantité de plutonium présente dans l'échantillon à partir de trois paramètres : le nombre total d'impulsions et les deux nombres de coïncidences d'ordre 2 déterminés à l'étape 302 ou à partir de cinq paramètres : les trois paramètres précités et les deux nombres de coïncidences d'ordre 3 déterminés à l'étape 303.

La figure 4a illustre, sur un exemple, le principe de détermination d'un nombre de coïncidences d'impulsions d'ordre N pour un train d'impulsions mesuré 400.

Selon un principe connu, notamment décrit dans la référence [5], une coïncidence d'impulsions d'ordre N existe lorsque, pour une impulsion donnée ayant un temps d'arrivée $t_0$, il existe N - 1 autres impulsions dans une fenêtre temporelle de taille donnée dont le début coïncide avec l'instant $t_0$. Le nombre N varie de 0 à $N_d$-1 où $N_d$ est le nombre de détecteurs du système.

Sur l'exemple de la figure 4a, la fenêtre temporelle a une taille de 100 ns et on a identifié les multiplicités d'ordre 0 ou coïncidences d'ordre 1 (aucune autre impulsion dans la fenêtre), multiplicités d'ordre 1 et d'ordre 2 (respectivement 1 et 2 impulsions supplémentaires dans la fenêtre).

Par ailleurs, on distingue les fenêtres R+A dites « réelles et accidentelles » qui sont appliquées à l'ensemble des impulsions mesurées, des fenêtres A dites « accidentelles » correspondant à des coïncidences accidentelles pour des fenêtres temporelles situées au-delà d'un certain décalage temporel qui est pris égal à environ 10 fois le temps de détection d'un neutron dans le système. Sur l'exemple de la figure 4a, ce décalage temporel est égal à 1000 ns.

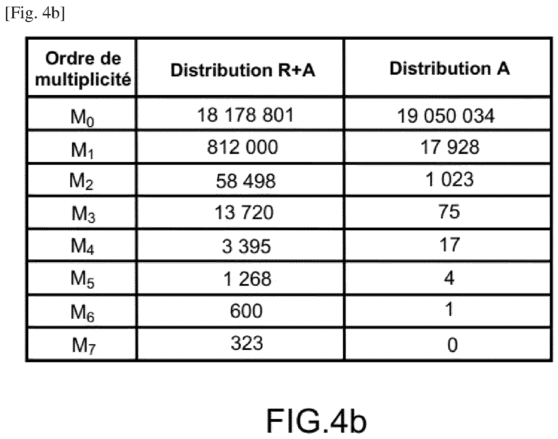

Pour déterminer le nombre de coïncidences d'ordre N, on détermine tout d'abord le nombre de multiplicités $M_N$ pour N variant de 0 à une valeur entière maximale max prédéfinie. On obtient une distribution des ordres de multiplicité donnée dans le tableau de la figure 4b pour l'exemple particulier de la figure 4a. A partir de cette distribution, on détermine des moments factoriels d'ordre k définis par la relation suivante :

$$(R + A)_k = \sum_{N=k}^{max} \frac{N!}{(N-k)!} M_{N\,(R+A)}$$

$$(A)_k = \sum_{N=k}^{max} \frac{N!}{(N-k)!} M_{N\,(A)}$$

Où :

(R+A)$_k$ et (A)$_k$ sont respectivement les moments factoriels d'ordre k des distributions (R+A) et (A)

$M_N$(R+A) et $M_N$(A) sont respectivement les ordres N des multiplicités des distributions (R+A) et (A),

« max » est l'ordre maximal de multiplicité qui est par exemple égal au nombre de détecteurs du système.

Les nombres de coïncidences réelles d'ordres 1,2 et 3 encore appelés singulets, doublets et triplets (S, D et T) sont alors donnés par les équations suivantes (voir références [5] [7]) :

$$S = (R + A)_0 = (A)_0$$

$$D = (R + A)_1 - (A)_1$$

$$T = \frac{1}{2}\left((R + A)_2 - (A)_2 - \frac{2A_1}{S}\left((R + A)_1 - (A)_1\right)\right)$$

S correspond au nombre total d'impulsions dans le train d'impulsions.

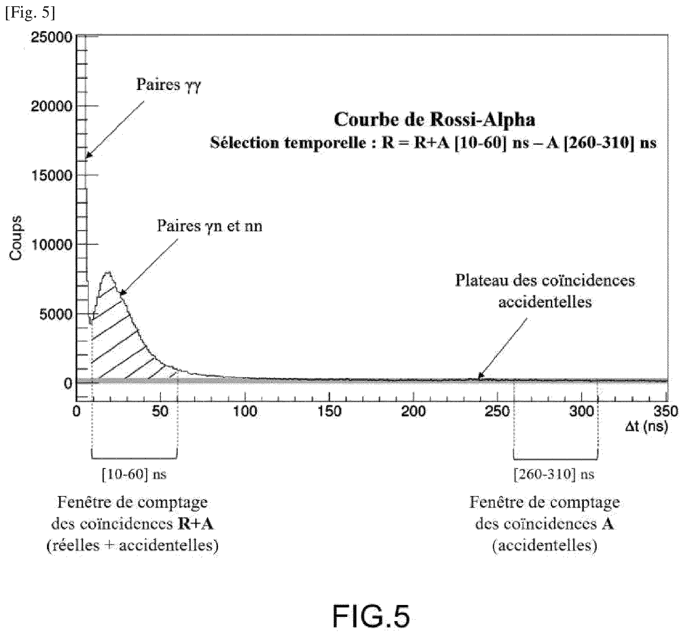

La figure 5 montre un exemple de courbe de Rossi-Alpha qui donne un histogramme des nombres de coïncidences d'impulsions d'ordre 2 en fonction de la valeur de la fenêtre temporelle.

Autrement dit, cet histogramme est obtenu en mesurant, pour chaque détection, le temps d'arrivée de l'impulsion suivante. L'exemple de la figure 5 est obtenu pour une source de plutonium $^{240}$Pu dans un système de détection composé de scintillateurs plastiques PVT.

Sur la courbe, on a identifié une première fenêtre temporelle [10-60] ns qui correspond au comptage des coïncidences réelles + accidentelles et une seconde fenêtre temporelle [260-310] ns qui correspond au comptage des coïncidences accidentelles.

Dans l'intervalle temporel [0-10] ns, sont concentrées les coïncidences d'ordre 2 correspondant aux paires $\gamma\gamma$. L'intervalle temporel [10-60] ns correspond aux paires $\gamma$n et nn.

Pour pouvoir quantifier précisément le plutonium en présence de curium, il est proposé d'exploiter deux intervalles temporels distincts pour comptabiliser le nombre de coïncidences d'impulsions d'ordre 2.

En effet, la présente invention est basée sur les différences entre les spectres en énergie des neutrons de fission spontanée du $^{244}$Cm et du $^{240}$Pu.

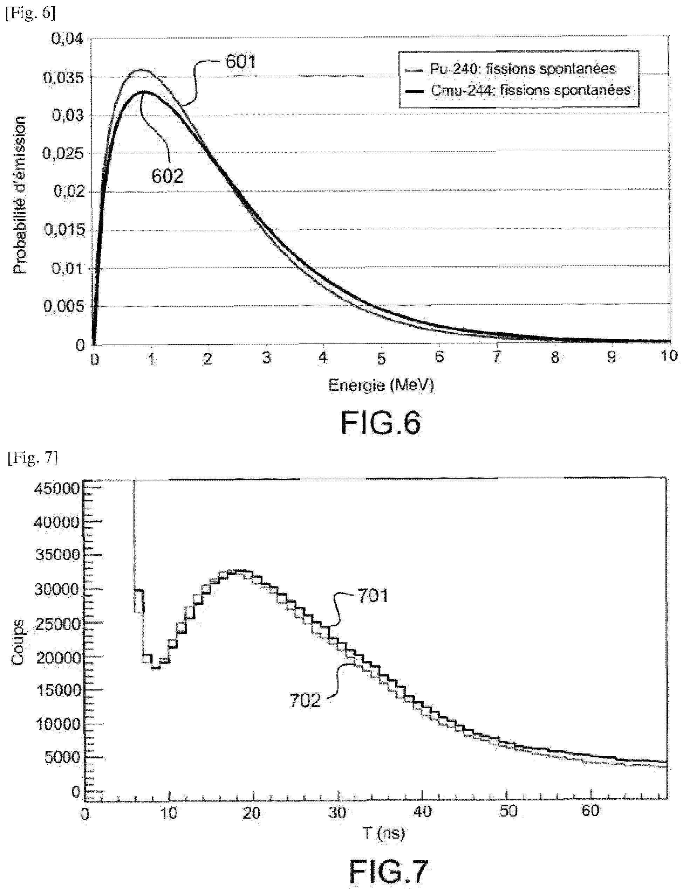

La figure 6 montre les spectres en énergie 601,602 des neutrons de fission spontanée du $^{240}$Pu et du $^{244}$Cm.

On peut voir sur cette figure une différence significative entre la probabilité d'émission du $^{240}$Pu (courbe 601) et du $^{244}$Cm (courbe 602) dans les plages d'énergie [0,2 - 2] MeV et [2-7] MeV.

La figure 7 représente une comparaison des courbes de Rossi-Alpha pour une même valeur de doublets mesurés sur une fenêtre temporelle [10-60] ns entre le $^{240}$Pu (701) et le $^{244}$Cm (702).

La figure 7 montre le temps moyen d'arrivée des neutrons et rayonnements gamma de fission suivant une première détection qui est généralement celle d'un rayonnement gamma du fait de leur vitesse supérieure (30 cm.ns$^{-1}$) à celle des neutrons (quelques cm.ns$^{-1}$, selon leur énergie).

On observe un premier pic correspondant à la détection de paires de rayonnements gamma, puis une « bosse » correspondant à celle de paires impliquant au moins un neutron, avec une allure comparable à celle des spectres en Figure 6 car la vitesse des neutrons varie comme la racine carrée de leur énergie. Il apparaît en Figure 7 une différence significative entre le $^{240}$Pu et le $^{244}$Cm en termes de nombres de coïncidences d'ordre 2 sur la fenêtre [20-60] ns, et également sur la fenêtre [10-20] ns. Ainsi, on observe une probabilité de détection des neutrons issus de la fission spontanée du $^{244}$Cm plus importante dans la fenêtre de coïncidences comprise entre 10 et 20 ns que pour du $^{240}$Pu, la tendance étant inversée entre 20 et 60 ns. En effet, les courbes 701,702 se croisent approximativement à l'instant t=20 ns.

Plus précisément, sur l'exemple numérique de la figure 7, les valeurs de nombres de coïncidences réelles d'ordre 2 sont données dans le tableau ci-dessous pour les deux intervalles temporels, respectivement pour un échantillon de plutonium et pour un échantillon de curium ayant chacun une masse équivalente à 400 g de $^{240}$Pu, pour un même nombre de coïncidences dans la fenêtre totale [10-60] ns.

|  | $^{240}$Pu (400 g) | $^{244}$Cm (400 g de $^{240}$Pu$_{éq}$) |
|---|---|---|
| D [10-20] ns | 286155 | 299059 |
| D [20-60] ns | 553343 | 535718 |

En exploitant cette différence de contribution sur deux fenêtres temporelles distinctes, il est possible d'améliorer la prédiction de la masse de $^{240}$Pu dans un échantillon en présence de $^{244}$Cm au moyen d'un modèle de régression non linéaire.

En effet, la prise en compte d'une première fenêtre temporelle dans laquelle la contribution du $^{240}$Pu est plus importante que celle du $^{244}$Cm et d'une seconde fenêtre temporelle dans laquelle la contribution du $^{244}$Cm est plus importante que celle du $^{240}$Pu comme paramètres d'un modèle de régression non linéaire permet d'améliorer la prédiction de la masse de $^{240}$Pu comme cela va être démontré par la suite.

La détermination précise de la frontière entre les deux fenêtres temporelles (ici égale à 20 ns) est faite par expérimentation en recherchant le meilleur compromis de performances, par exemple en testant différentes fenêtres temporelles et en comparant les prédictions du modèle de régression obtenu.

Par exemple, les deux fenêtres temporelles peuvent être définies telles que le ratio des nombres de coïncidences respectifs des deux éléments $^{240}$Pu et $^{244}$Cm est supérieur à un premier seuil prédéfini pour l'une des fenêtres et inférieur à un second seuil prédéfini pour l'autre fenêtre. Dans l'exemple ci-dessus, le ratio entre les contributions de $^{244}$Cm et $^{240}$Pu vaut 1,04 pour la fenêtre [10-20] ns et 0,97 pour la fenêtre [20-60] ns.

L'étape 304 du procédé selon l'invention consiste à exécuter un modèle de régression non linéaire préalablement entrainé pour déterminer une masse ou une quantité de $^{240}$Pu à partir de plusieurs paramètres de nombres de coïncidences.

Le modèle est par exemple un modèle de régression multilinéaire des moindres carrés ou un réseau de neurones artificiels tel que décrit dans la référence [11] ou plus généralement tout modèle apprenant qui peut être entrainé pour prédire une quantité de plutonium à partir de plusieurs mesures de nombres de coïncidences d'impulsions.

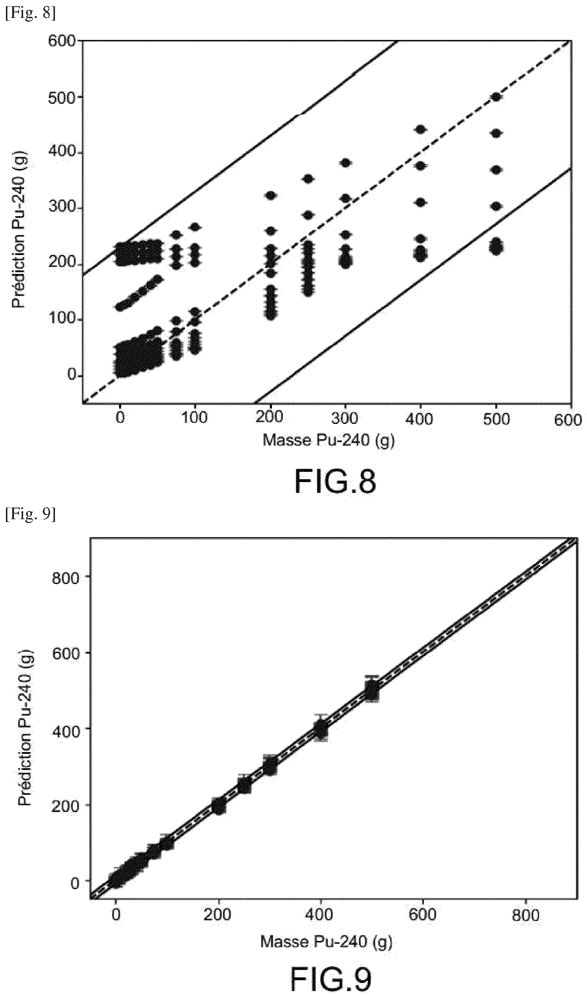

Les figures 8, 9 et 10 illustrent des résultats de prédiction obtenus avec un modèle de régression non linéaire basé sur une méthode de Monte Carlo pour des données simulées correspondant à une source composée de plutonium et de curium.

Le nombre de configurations correspond à un plan d'expérience factoriel complet qui est composé de trois paramètres variables, à savoir les masses de $^{244}$Cm et de $^{240}$Pu, l'activité d'une source de $^{60}$Co (émettrice de deux rayonnements gamma corrélés à 1173 keV et 1332 keV). Un plan factoriel complet comprenant un grand nombre de configurations des valeurs précitées est utilisé comme base d'apprentissage pour l'entrainement du modèle de prédiction. Une fois le modèle entrainé sur cette base, il peut être utilisé pour estimer une masse de $^{240}$Pu à partir de nouvelles mesures réalisées sur un nouvel échantillon inconnu.

Les données d'entrainement donnent a minima les masses de $^{244}$Cm et de $^{240}$Pu, ainsi que les valeurs des variables explicatives du modèle (nombre total d'impulsions, nombres de coïncidences dans deux intervalles distincts). Les données d'entrainement peuvent être mesurées au moyen du dispositif décrit aux figures 1 et 2 sur plusieurs échantillons ou peuvent être simulées avec un code de transport de particules basé sur la méthode de Monte Carlo comme celui décrit dans la référence [9].

La figure 8 montre, sur un diagramme, les résultats de prédiction (en ordonnée) en fonction de la valeur réelle de la masse de $^{240}$Pu pour un modèle de régression prenant comme entrées uniquement le nombre total d'impulsions (encore appelé singulets S) et le nombre de coïncidences d'impulsions d'ordre 2 compté dans une seule fenêtre temporelle égale à [10-60] ns (encore appelé doublets D).

Comme on peut le voir sur la figure 8, les résultats de prédiction sont très dispersés et peu conformes aux valeurs réelles. Cela est dû au fait que le modèle n'arrive pas à discriminer la contribution du plutonium de celle du curium dans la fenêtre complète [10-60] ns dans laquelle le $^{244}$Cm et le $^{240}$Pu présentent le même nombre de coïncidences d'ordre 2.

La figure 9 montre les résultats de prédiction lorsque le modèle prend cette fois en entrée trois paramètres : le nombre total d'impulsions, un premier nombre de coïncidences réelles d'impulsions d'ordre 2 compté dans une première fenêtre temporelle égale à [10-20] ns et un second nombre de coïncidences réelles d'impulsions d'ordre 2 compté dans une seconde fenêtre temporelle égale à [10-60] ns.

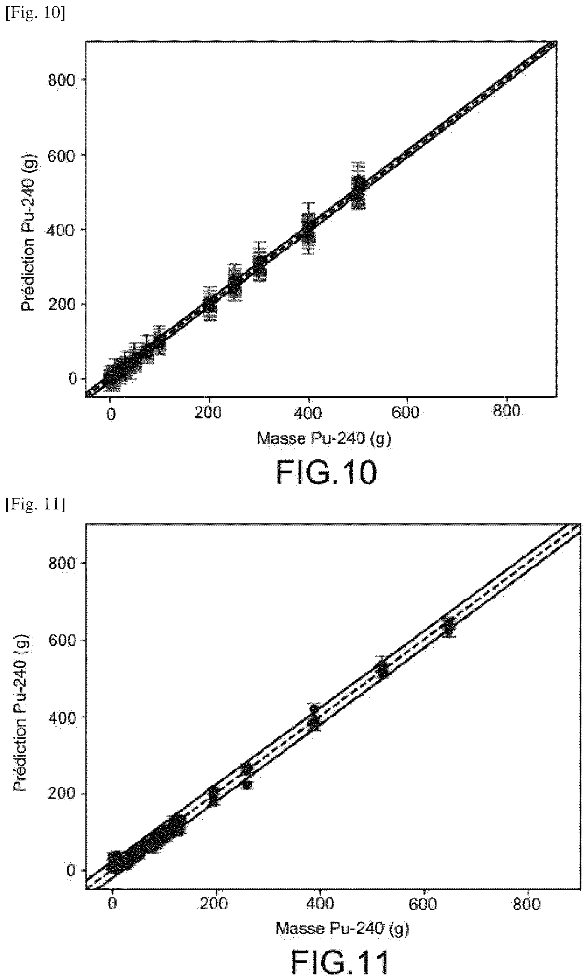

La figure 10 montre, alternativement, les résultats de prédiction lorsque le modèle prend en entrée trois paramètres : le nombre total d'impulsions, un premier nombre de coïncidences réelles d'impulsions d'ordre 2 compté dans une première fenêtre temporelle égale à [20-60] ns et un second nombre de coïncidences réelles d'impulsions d'ordre 2 compté dans une seconde fenêtre temporelle égale à [10-60] ns.

Les figures 9 et 10 montrent que la prise en compte dans le modèle de régression des trois variables explicatives que sont les singulets d'une part et les doublets comptés dans deux fenêtres de coïncidences distinctes comme D [10-20] ns et D [10-60] ns (voir Figure 9), ou D [20-60] ns et D [10-60] ns (voir Figure 10), d'autre part, permet d'obtenir une prédiction nettement meilleure de la masse de $^{240}$Pu qu'en utilisant une seule fenêtre D [10-60] ns (figure 8).

Les valeurs prédites sont en effet beaucoup plus proches de la valeur réelle en Figures 9 ou 10 qu'en Figure 8, et ce quelle que soit la quantité de curium. Ce résultat est lié à la séparation des variables explicatives en deux fenêtres distinctes, une fenêtre globale et une fenêtre dans laquelle l'influence du plutonium ou du curium est plus importante.

Les résultats illustrés aux figures 8 à 10 sont obtenus avec des données simulées pour des sources ponctuelles de plutonium et de curium situées au centre du fût de 118 L rempli de cellulose de densité 0,3. Afin d'évaluer l'impact lié à l'effet de multiplication par fissions induites, qui devient significatif pour les masses de plutonium supérieures à 100 g, un second plan d'expériences à 124 configurations est simulé pour le cas d'une sphère de poudre de $PuO_2$ de densité 3, toujours située au centre de la même matrice organique.

Les variables simulées pour ce second plan d'expériences sont le volume de la sphère de poudre de $PuO_2$, liée à la masse totale de plutonium, la masse de $^{240}$Pu et la masse de $^{244}$Cm.

La figure 11 montre les résultats de prédiction obtenus avec le même modèle de régression prenant comme entrée le nombre total d'impulsions, un premier nombre de coïncidences d'impulsions d'ordre 2 compté dans une première fenêtre temporelle égale à [10-20] ns et un second nombre de coïncidences d'impulsions d'ordre 2 compté dans une seconde fenêtre temporelle égale à [10-60] ns.

Même si l'on observe à la figure 11 une dégradation des performances du modèle de régression par rapport aux résultats des figures 9 et 10 pour les masses de $^{240}$Pu équivalent supérieures à 100 g, en raison de l'effet de multiplication, les performances du modèle composé des 3 variables explicatives précitées S, D [10-20] ns, D [10-60] ns restent néanmoins tout à fait acceptables pour une application pratique.

De façon plus générale, les valeurs numériques des intervalles temporels de comptage des doublets dépendent du dispositif de détection considéré ainsi que de la distance entre l'échantillon et les détecteurs.

Les intervalles à prendre en compte comme entrées du modèle de régression sont plus généralement choisis comme deux intervalles temporels contigus dans lesquels respectivement l'un des deux éléments parmi le $^{240}$Pu ou le $^{244}$C$_m$ contribue plus que l'autre et inversement.

On décrit à présent un autre mode de réalisation de l'invention pour lequel le modèle de régression prend en entrée 5 paramètres : les 3 paramètres S, D [10-20] ns, D [10-60] ns décrits précédemment et 2 autres paramètres qui sont un premier nombre de coïncidences d'impulsions d'ordre 3 compté dans une première fenêtre temporelle égale à [10-20] ns, et un second nombre de coïncidences d'impulsions d'ordre 3 compté dans une seconde fenêtre temporelle égale à [10-60] ns.

Un modèle de régression composé de ces 5 variables explicatives permet de limiter fortement l'influence de l'effet de multiplication (significatif pour des masses de plutonium supérieures à 100 g) sur l'estimation de la masse de plutonium.

En effet, l'utilisation de ces coïncidences d'ordre 3 permet de mieux exploiter la différence de multiplicité neutronique entre le $^{244}$Cm et le $^{240}$Pu. Il est ainsi possible d'observer une meilleure prédiction de la masse de $^{240}$Pu avec des modèles de régression constitués de 5 variables explicatives évaluées dans une fenêtre temporelle comprise entre 10 et 20 ns et une autre comprise entre 10 et 60 ns.

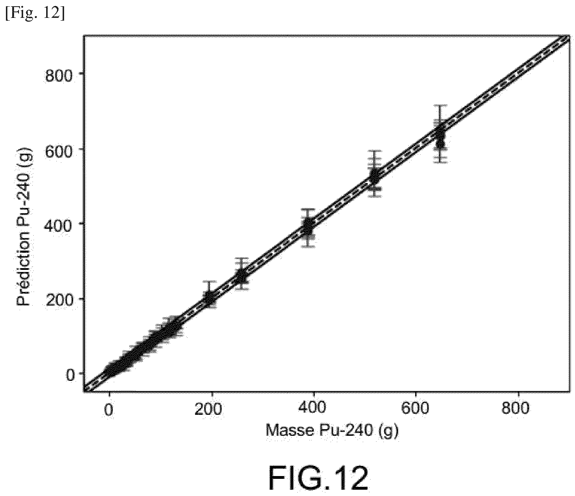

La figure 12 montre les résultats de prédiction obtenus pour un modèle constitué des 5 variables précitées.

Dans une variante de réalisation, les nombres de coïncidences d'impulsions d'ordre 3 peuvent être déterminés au moyen d'un histogramme en deux dimensions des coïncidences d'ordre 3.

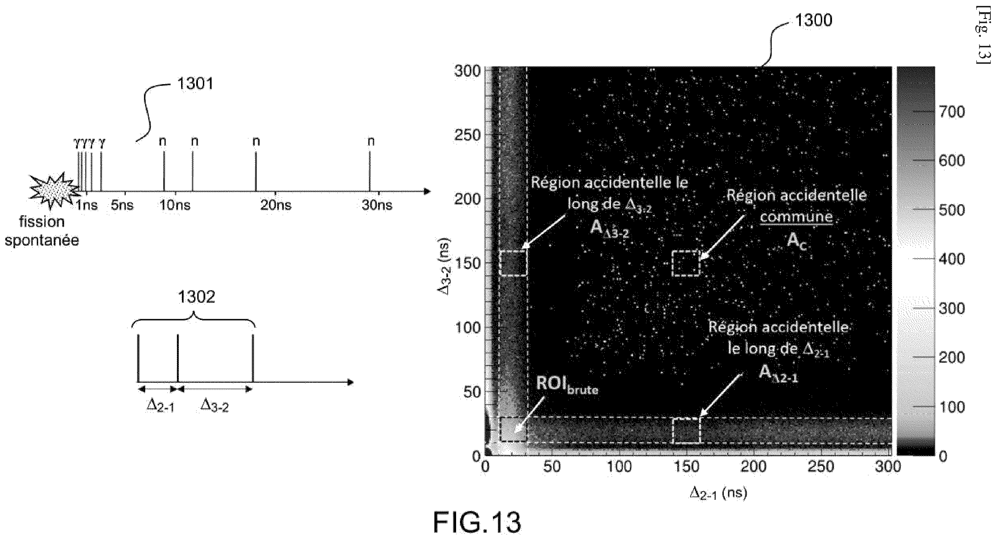

La figure 13 illustre un tel histogramme 1300 construit à partir d'un train d'impulsions 1301. L'histogramme représente toutes les combinaisons de 3 impulsions coïncidentes 1302 dont l'intervalle temporel entre les deux premières impulsions est reporté sur l'axe $\Delta_{2\text{-}1}$ et l'intervalle temporel entre la 2$^{ième}$ et la 3$^{ième}$ impulsion est reporté sur l'axe $\Delta_{3\text{-}2}$.

Le nombre de coïncidences d'impulsions d'ordre 2 peut être déterminé en sommant les valeurs d'une zone d'intérêt de l'histogramme ROI$_{nette}$ qui est définie par les fenêtres temporelles souhaitées.

Selon la référence [3] cette zone d'intérêt est donnée par la relation :

$$\text{ROI}_{nette} = (\text{ROI}_{brute} - A_c) - (A_{\Delta 2\text{-}1} - A_C) - (A_{\Delta 3\text{-}2} - A_C)$$

$A_C$ est la région accidentelle commune de l'histogramme 1300

$A_{\Delta 2\text{-}1}$ est la région accidentelle le long du premier axe de l'histogramme

$A_{\Delta 3\text{-}2}$ est la région accidentelle le long du second axe de l'histogramme

ROI$_{brute}$ correspond à la zone de comptage dans la fenêtre temporelle donnée.

Ainsi, de façon similaire à ce qui a été décrit précédemment, les deux variables explicatives de nombres de coïncidences d'ordre 3 déterminées pour les deux fenêtres temporelles [10-20] ns et [10-60] ns peuvent être déterminées via le calcul de ROI$_{nette}$ sur cet histogramme 1300.

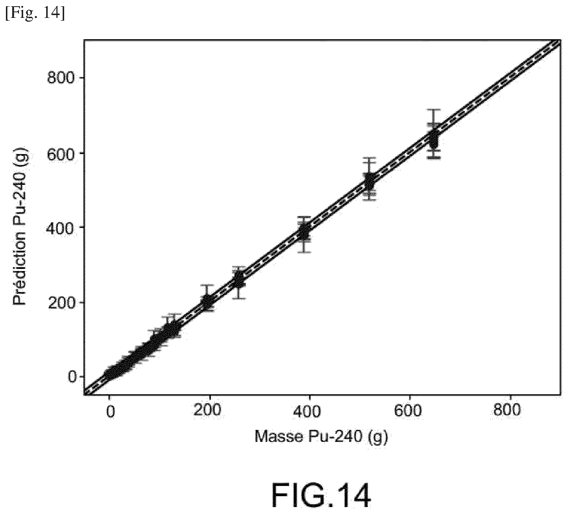

La figure 14 montre les résultats de prédiction obtenus pour un modèle constitué des 5 variables en calculant les nombres de coïncidences d'ordre 3 au moyen de l'histogramme de la figure 13.

En résumé, le modèle de régression non linéaire exécuté à l'étape 304 du procédé selon l'invention peut être constitué de 3 ou 5 variables explicatives.

Les trois premières variables sont :

- le nombre total d'impulsions qui correspond au nombre de coïncidences d'ordre 1 (singulets),

- un premier nombre de coïncidences d'ordre 2 (doublets) calculé dans une première fenêtre temporelle choisie telle que la contribution du plutonium est supérieure ou inférieure à celle du curium,

- un premier nombre de coïncidences d'ordre 2 (doublets) calculé dans une deuxième fenêtre temporelle qui correspond à la concaténation de la première fenêtre temporelle et d'une troisième fenêtre temporelle telle que les contributions respectives du plutonium ou du curium soient inversées par rapport à la première fenêtre temporelle.

Les deux variables supplémentaires sont :

- un premier nombre de coïncidences d'ordre 3 (triplets) calculé dans la première fenêtre temporelle,

- un premier nombre de coïncidences d'ordre 3 (triplets) calculé dans la deuxième fenêtre temporelle

- le nombre de coïncidences d'ordre 3 pouvant être calculé directement à partir du train d'impulsions (triplets) ou via la construction d'un histogramme en deux dimensions (ROI$_{nette}$).

Par exemple, la première fenêtre temporelle est [10-20] ns, la deuxième fenêtre temporelle est [10-60] ns, la troisième fenêtre temporelle est [20-60]ns.

Alternativement, la première fenêtre temporelle est [20-60] ns, la deuxième fenêtre temporelle est [10-60] ns, la troisième fenêtre temporelle est [10-20]ns.

Références :

[1] D. H. Beddingfield, A. P. Belian, « Detection of Cm-244 in Plutonium-Bearing Wastes at reprocessing Facilities », Los Alamos National Laboratory, 2004

[2] V. Bottau, R. De Stefano, C. Carasco, C. Eleon, B. Perot, « Procédé de détection de radiations et système associé mettant e œuvre une discrimination des coïncidences neutron-gamma », demande de brevet EP 3 835 831 A1.

[3] V. Bottau, C. Carasco, B. Pérot, C. Eleon, R. De Stefano, L. Isnel, I. Tsekhanovich, « Detection of Fission Coincidences With Plastic Scintillators for the Characterization of Radioactive Waste Drums », TNS IEEE, volume 69, issue 4, 2022

[4] N. Ensslin, « Passive Nondestructive Assay of Nuclear Materials, », 1991

[5] J-B Porcher, T. Lambert, N. Saurel, H. Schoech, L. Tondut, C. Passard, G. Granier « Dossier de Recommandations pour l'Optimisation des Mesures Neutroniques Passives », Rapport CEA, 2013, ISSN 0429-3460,

[7] N. Ensslin, "Application Guide to Neutron Multiplicity Counting", LA-13442-M, UC-700, November 1998.

[8] Patrick M J Chard et al, "Field Examples of Waste Assay Solutions for Curium-Contaminated Wastes", ICEM2009-16259

[9] S. A. Pozzi, E. Padovani, M. Marseguerra, "MCNP-PoliMi, a Monte-Carlo code for correlation measurements", Nuclear Instruments and Methods in Physics Research, A 513 (2003) 550-558.

[10] B. Perot et al, "The characterization of radioactive waste: a critical review of techniques implemented or under development at CEA, France", EPJ Nuclear Sci. Technol. Volume 4, 2018

[11] Pedregosa, et al., "Scikit-learn: Machine learning in Python." Journal of Machine Learning Research, 2825-2830, 2011.

**Revendications**

**1.** Procédé de détermination d'une quantité de plutonium dans un échantillon radioactif en présence de curium, le procédé comprenant les étapes de :

- Mesurer (301) un train d'impulsions électriques au moyen d'un système de détection de radiations comprenant plusieurs détecteurs disposés autour de l'échantillon et aptes à générer une impulsion électrique en réponse à la détection d'un rayonnement,

- Déterminer (302), respectivement dans une première et dans une seconde fenêtres temporelles, un premier et un second nombre de coïncidences d'impulsions d'ordre 2 entre les impulsions électriques fournies par les détecteurs,
- La seconde fenêtre temporelle étant constituée de l'union de la première fenêtre temporelle et d'une troisième fenêtre temporelle,
- La première fenêtre temporelle et la troisième fenêtre temporelle étant choisies de manière à ce que le nombre de coïncidences d'impulsions d'ordre 2 liées au plutonium soit plus important que celui lié au curium dans l'une des deux fenêtres parmi la première fenêtre temporelle et la troisième fenêtre temporelle et moins important que celui lié au curium dans l'autre fenêtre,
- Déterminer (304) la quantité de plutonium en appliquant un modèle de régression non linéaire prenant comme paramètres : le nombre total d'impulsions mesurées, le premier nombre de coïncidences d'impulsions d'ordre 2 et le second nombre de coïncidences d'impulsions d'ordre 2, le modèle étant au préalable entrainé sur un ensemble de valeurs simulées à partir d'un modèle numérique.

2. Procédé de détermination d'une quantité de plutonium selon la revendication 1 dans lequel la seconde fenêtre temporelle est choisie de manière à inclure des paires d'impulsions correspondant à des rayonnements de type $(\gamma,n)$ et $(n,n)$ et d'exclure des paires d'impulsions correspondant à des rayonnements de type $(\gamma,\gamma)$.

3. Procédé de détermination d'une quantité de plutonium selon l'une quelconque des revendications précédentes comprenant en outre les étapes de :

- Déterminer (303), respectivement dans la première et dans la seconde fenêtre temporelle, un premier et un second nombre de coïncidences d'impulsions d'ordre 3 entre les impulsions électriques fournies par les détecteurs,
- Le modèle de régression non linéaire prenant en outre comme paramètres lesdits premier et second nombres de coïncidences d'impulsions d'ordre 3

4. Procédé de détermination d'une quantité de plutonium selon l'une quelconque des revendications 1 ou 2 comprenant en outre les étapes de :

- Déterminer (303), un premier nombre de coïncidences d'impulsions d'ordre 3 entre les impulsions électriques fournies par les détecteurs qui est tel que l'écart temporel entre la première et la deuxième impulsions est compris dans la première fenêtre temporelle, et l'écart temporel entre la deuxième et la troisième impulsion est compris dans la première fenêtre temporelle,
- Déterminer (303), un second nombre de coïncidences d'impulsions d'ordre 3 entre les impulsions électriques fournies par les détecteurs qui est tel que l'écart temporel entre la première et la deuxième impulsions est compris dans la deuxième fenêtre temporelle et l'écart temporel entre la deuxième et la troisième impulsion est compris dans la deuxième fenêtre temporelle,
- Le modèle de régression non linéaire prenant en outre comme paramètres lesdits premier et second nombres de coïncidences d'impulsions d'ordre 3

5. Procédé de détermination d'une quantité de plutonium selon l'une quelconque des revendications 3 ou 4 dans lequel la seconde fenêtre temporelle est choisie de manière à inclure des triplets d'impulsions correspondant à des rayonnements de type (y,n,n) et (n,n,n) et à exclure des triplets d'impulsions correspondant à des rayonnements de type (y,y,y).

6. Procédé de détermination d'une quantité de plutonium selon l'une quelconque des revendications précédentes dans lequel la première fenêtre temporelle est égale à [10 ns ; 20 ns], la deuxième fenêtre temporelle est égale à [10 ns ; 60 ns] et la troisième fenêtre temporelle est égale à [20 ns ; 60 ns].

7. Procédé de détermination d'une quantité de plutonium selon l'une quelconque des revendications 1 à 5 dans lequel la première fenêtre temporelle est égale à [20 ns ; 60 ns], la deuxième fenêtre temporelle est égale à [10 ns ; 60 ns] et la troisième fenêtre temporelle est égale à [10ns ; 20 ns].

8. Système de détermination d'une quantité de plutonium dans un échantillon radioactif en présence de curium, le système comprenant plusieurs détecteurs disposés autour de l'échantillon et aptes à générer une impulsion électrique en réponse à la détection d'un rayonnement et un calculateur configuré pour exécuter les étapes du procédé selon l'une quelconque des revendications précédentes.

9. Système de détermination d'une quantité de plutonium selon la revendication 8 dans lequel chaque détecteur comprend un scintillateur organique plastique en polyvinyle-toluène (PVT).

[Fig. 1]

FIG.1

[Fig. 2]

FIG.2

[Fig. 3]

FIG.3

[Fig. 4a]

FIG.4a

[Fig. 4b]

| Ordre de multiplicité | Distribution R+A | Distribution A |
|:---:|:---:|:---:|
| $M_0$ | 18 178 801 | 19 050 034 |
| $M_1$ | 812 000 | 17 928 |
| $M_2$ | 58 498 | 1 023 |
| $M_3$ | 13 720 | 75 |
| $M_4$ | 3 395 | 17 |
| $M_5$ | 1 268 | 4 |
| $M_6$ | 600 | 1 |
| $M_7$ | 323 | 0 |

# FIG.4b

[Fig. 5]

FIG.5

[Fig. 6]

FIG.6

[Fig. 7]

FIG.7

[Fig. 8]

FIG.8

[Fig. 9]

FIG.9

[Fig. 10]

FIG.10

[Fig. 11]

FIG.11

[Fig. 12]

FIG.12

[Fig. 13]

FIG.13

[Fig. 14]

FIG.14

Europäisches Patentamt
European Patent Office
Office européen des brevets

# RAPPORT DE RECHERCHE EUROPEENNE

Numéro de la demande

EP 25 18 0791

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | PÉROT BERTRAND ET AL: "The characterization of radioactive waste: a critical review of techniques implemented or under development at CEA, France", EPJ N - NUCLEAR SCIENCES & TECHNOLOGIES , vol. 4 1 janvier 2018 (2018-01-01), XP055857404, DOI: 10.1051/epjn/2017033 Extrait de l'Internet: URL:https://hal-cea.archives-ouvertes.fr/cea-01794043/document * alinéa [0004] - alinéa [0007] * * abrégé; figures * ----- | 1-9 | INV. G01T1/167 G21C17/06 |
| A | FR 3 118 494 A1 (COMMISSARIAT ENERGIE ATOMIQUE [FR]) 1 juillet 2022 (2022-07-01) * alinéa [0111] * ----- | 1-9 | |
| A | BEDDINGFIELD D H ET AL: "Detection of Cm-244 in Plutonium-Bearing Wastes at Reprocessing Facilities", TRANSACTIONS OF THE AMERICAN NUCLEAR SOCIETY., vol. 95, no. 1, 1 novembre 2006 (2006-11-01), pages 47-49, XP093240396, US ISSN: 0003-018X * le document en entier * ----- | 1-9 | |

DOMAINES TECHNIQUES RECHERCHES (IPC)

G01T
G21C

Le présent rapport a été établi pour toutes les revendications

| Lieu de la recherche | Date d'achèvement de la recherche | Examinateur |
|---|---|---|
| La Haye | 17 octobre 2025 | Eberle, Katja |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...............................................................................
& : membre de la même famille, document correspondant

EPO FORM 1503 03.82 (P04C02)

**EP 4 667 977 A1**

## ANNEXE AU RAPPORT DE RECHERCHE EUROPEENNE
## RELATIF A LA DEMANDE DE BREVET EUROPEEN NO.

EP 25 18 0791

17-10-2025

| Document brevet cité au rapport de recherche | Date de publication | Membre(s) de la famille de brevet(s) | Date de publication |
|---|---|---|---|
| FR 3118494 A1 | 01-07-2022 | EP 4267993 A1 | 01-11-2023 |
| | | FR 3118494 A1 | 01-07-2022 |
| | | WO 2022136618 A1 | 30-06-2022 |

EPO FORM P0460

## RÉFÉRENCES CITÉES DANS LA DESCRIPTION

**Documents brevets cités dans la description**

- EP 3835831 A1

**Littérature non-brevet citée dans la description**

- **D. H. BEDDINGFIELD** ; **A. P. BELIAN**. Detection of Cm-244 in Plutonium-Bearing Wastes at reprocessing Facilities. Los Alamos National Laboratory, 2004
- **V. BOTTAU** ; **C. CARASCO** ; **B. PÉROT** ; **C. ELEON** ; **R. DE STEFANO** ; **L. ISNEL** ; **I. TSEKHANOVICH**. Detection of Fission Coincidences With Plastic Scintillators for the Characterization of Radioactive Waste Drums. *TNS IEEE*, vol. 69 (4)
- **N. ENSSLIN**. *Passive Nondestructive Assay of Nuclear Materials*, 1991
- **J-B PORCHER** ; **T. LAMBERT** ; **N. SAUREL** ; **H. SCHOECH** ; **L. TONDUT** ; **C. PASSARD** ; **G. GRANIER**. Dossier de Recommandations pour l'Optimisation des Mesures Neutroniques Passives. *Rapport CEA*, 2013, ISSN 0429-3460
- **N. ENSSLIN**. Application Guide to Neutron Multiplicity Counting. *LA-13442-M, UC-700*, November 1998
- **PATRICK M J CHARD et al.** Field Examples of Waste Assay Solutions for Curium-Contaminated Wastes. *ICEM2009-16259*
- **S. A. POZZI** ; **E. PADOVANI** ; **M. MARSEGUERRA**. MCNP-PoliMi, a Monte-Carlo code for correlation measurements. *Nuclear Instruments and Methods in Physics Research, A*, 2003, vol. 513, 550-558
- **B. PEROT et al.** The characterization of radioactive waste: a critical review of techniques implemented or under development at CEA, France. *EPJ Nuclear Sci. Technol.*, 2018, vol. 4
- **PEDREGOSA et al.** Scikit-learn: Machine learning in Python. *Journal of Machine Learning Research*, 2011, 2825-2830